# EUROPEAN PATENT APPLICATION

(11) **EP 4 664 224 A2**
(43) Date of publication of application: **17.12.2025**
(21) Application number: 25211365.9
(22) Date of filing: 13.09.2021
(51) Int. Cl.: G05D 1/00

(54) **FLOOR SAWING EQUIPMENT WITH CONTROLLABLE SUPPORTING WHEELS**

(30) Priority: 24.09.2020 SE 2051110; 24.09.2020 SE 2051111; 24.09.2020 SE 2051112; 24.09.2020 SE 2051113; 24.09.2020 SE 2051114
(62) Divisional of application: 21873055.4
(71) Applicant: Husqvarna AB, 561 82 Huskvarna (SE)
(72) Inventor: Gustafsson, Tobias, Göteborg (SE); Sandwall, Johan, Gråbo (SE)

(57) **Abstract**

A floor saw (100, 100a, 100b) comprising a control unit (130) and an arrangement configured to determine a weight and/or volume of accumulated dust or slurry in a dust container (180) mounted on the floor saw, where the control unit (130) is arranged to detect when the weight and/or volume of accumulated dust or slurry exceeds a preconfigured threshold and to transmit a signal to a remote control device (310) indicating that the weight and/or volume of accumulated dust or slurry is above the preconfigured threshold.

## Description

### TECHNICAL FIELD

The present disclosure relates to concrete processing and in particular to floor saws for sawing in a concrete surface segment. There are disclosed floor saws, systems and methods which allow for mitigating floor saw drifting due to a cutting blade mounted offset to a side of the floor saw.

### BACKGROUND

Early entry concrete sawing is a concrete processing technique where shallow cuts, often in straight lines, are made in concrete surfaces within the first one or two hours after finishing surface preparation, i.e., after the concrete has reached a certain level of maturity but before the concrete has set completely, a period often referred to as the 'green zone'. It is not always easy to predict when this period is to occur. The shallow cuts create a weakened surface plane, thus encouraging cracking to occur at the location of the cuts in a controlled manner rather than the cracks appearing anywhere in the concrete, resulting in a more visually appealing surface. One example of early entry concrete saws are the Soff-Cut range of early entry concrete saw products manufactured by Husqvarna AB.

Many floor saws for sawing in concrete surfaces are arranged with a saw blade that is offset to one side of a center line of the saw, i.e., offset transversally to the cutting direction of the floor saw. This offset allows, e.g., for making cuts close to a wall or other obstacle. The offset, however, creates an imbalance in the saw which often results in the saw drifting towards the side where the saw blade is mounted. Some different ways of compensating for this 'natural' drifting in concrete saws are known. For instance, different fixed gear ratios on the left and right driven wheels can be used to bias the drive and thereby counter the drift. The entire driven axle on the floor saw can also be steered, i.e., adjusted, to compensate for the drift. These methods of compensating for the drift are seldom perfect. Thus, it is often seen in field that the operator of the floor saw manually compensates for residual drift by body weight, which is not ideal since it makes the concrete processing operation more difficult and places additional strain on the operator.

Floor saws may also be referred to as flat saws or road saws.

There is a need for improved floor saws which enable a more efficient and convenient concrete surface sawing operation.

### SUMMARY

It is an object of the present disclosure to provide improved floor saws. The object is at least in part obtained by a floor saw for sawing in a concrete surface segment according to claim 1.

The disclosed floor saw comprises a control unit and an arrangement configured to determine a weight and/or volume of accumulated dust or slurry in a dust container mounted on the floor saw. The control unit is arranged to detect when the weight and/or volume of accumulated dust or slurry exceeds a preconfigured threshold and to transmit a signal to a remote control device of the floor saw indicating that the weight and/or volume of accumulated dust or slurry is above the preconfigured threshold. Thus, advantageously, the operator does not have to monitor the dust container regularly to see if there is enough dust and slurry to merit emptying the dust container.

The floor saw normally comprises a circular cutting blade arranged transversally offset from a centrum line of the floor saw, which centrum line is aligned with a forward direction of the floor saw. The floor saw may further comprise a first drive wheel and a second drive wheel separated by the centrum line and arranged to support the floor saw on the concrete surface segment, where the drive wheels are arranged to be driven by respective first and second electric machines. The control unit can be arranged to control a difference between wheel forces of the drive wheels by controlling a respective torque and/or speed of the first and/or second electric machines in dependence of a desired yaw motion by the floor saw.

Thus, drifting by the floor saw due to the offset cutting blade can be compensated for by the control unit varying the wheel force at the drive wheels. The drift compensation systems presented herein can be used with a cutting blade offset to any side of the centrum line, i.e., a cutting blade mounted on the left side or the right side of the cutting machine. Since the yaw motion can be continuously or at least regularly sensed, drift compensation can easily be automatically updated as the concrete surface segment matures and becomes more hard. An operator can also control yaw motion conveniently by an input device such as a remote control or a control handle on the machine. The control input can advantageously be limited in dependence of the saw blade specifications so as to not exert too high strain on the saw blade.

The floor saws discussed herein are particularly suitable for use in early entry concrete surface sawing, but can also be used for more general floor and/or road or pavement sawing operations.

According to aspects, the floor saw comprises a first supporting wheel arranged on an opposite side of the centrum line compared to the circular cutting blade. This first supporting wheel comprises a wheel brake arranged to be controlled by the control unit to generate a negative wheel torque. Thus, it is appreciated that drift compensation can be performed also by braking one wheel. This method of compensating for drift is particularly suitable if both wheels are driven by the same motor at constant torque or speed, such as by the motor driving the cutting blade of the floor saw.

According to aspects, the floor saw comprises a weight and an actuator arranged to move the weight transversal to the forward direction. The control unit is then arranged to control the actuator to reduce a difference between the current yaw motion of the floor saw and the desired yaw motion setting. By varying the position of the weight, the normal forces acting on the supporting wheels are redistributed. The resulting change in friction for the wheels will have an impact on the wheel forces which are possible to generate in the forward or longitudinal direction, and thus the position of the weight can be used for drift compensation in a low complex manner even if the wheels are driven by the same constant torque motor.

According to aspects, the sensor arrangement comprises an inertial measurement unit (IMU) configured to detect the current yaw motion by the floor saw. The IMU is able to detect acceleration, which in turn can be used to estimate the yaw motion in a cheap and reliable manner. The IMU is optionally arranged distally mounted on a guiding arm extending in the forward direction. This distal mounting amplifies the acceleration by the leverage effect it provides and therefore simplifies measurement of acceleration.

According to aspects, the sensor arrangement comprises one or more vision-based sensors configured to detect the current yaw motion by the floor saw by changes in a forward direction view over time. Vision-based sensors can provide a highly accurate estimation of the yaw motion, which is an advantage. The type of vision-based sensors required for yaw motion estimation can be of relatively low resolution, and therefore also of low cost, which is an advantage.

According to aspects, the sensor arrangement comprises a receiver configured to receive a wireless signal from a beacon and to detect the current yaw motion by the floor saw and/or a current heading of the floor saw based on the received wireless signal. The beacon can be deployed by an operator and the floor saw can be made to saw along a line towards the beacon. This is a way of providing a floor saw with a degree of autonomy, which is an advantage since the operator may not necessarily need to constantly monitor the progress of the floor saw. Even if an operator is required, the operator can obtain guiding support from the deployed beacon, and also an improved drift compensation, thus providing for a more convenient floor sawing operation.

According to aspects, the sensor arrangement comprises an electronic compass configured to measure the forward direction of the floor saw in relation to magnetic north, wherein the control unit is configured to determine the current yaw motion of the floor saw based on measurements of the forward direction in relation to magnetic north over time. Electronic compasses are both low cost and reliable sources for yaw motion estimation. Disturbances in Earths magnetic field have little effect on the drift compensation, since the resulting magnetic compass deviation does not affect the relative compass measurements used for yaw motion estimation.

According to aspects, the control unit is arranged to receive the desired yaw motion setting via wireless radio link from a remote control device. This means that the drift prevention system can be used also as a remote control system. By indicating a desired yaw motion setting the floor saw can be made to follow a desired path, including turning maneuvers. For instance, the remote control device can be configured to transmit a desired yaw motion setting corresponding to sawing along a straight line, or corresponding to sawing along an arcuate curve having a configurable radius, or corresponding to a path with a deviation defined by a number of degrees from a set course after a controlled turning maneuver. Optionally, the remote control device can be configured to control one or more further floor saws in addition to the floor saw. This allows a single operator to control more than one saw, which reduces the need for operators and/or allows an operator to produce more floor saw cuts in a fixed amount of time compared to if that operator only controls a single floor saw.

According to aspects, the control unit is arranged to determine a level of drift compensation applied via the at least two supporting wheels to reduce the difference between the current yaw motion of the floor saw and the desired yaw motion setting. The control unit is in this case arranged to determine a concrete hardness level in dependence of the determined level of drift compensation, and to configure a desired speed of the floor saw in the forward direction in dependence of the concrete hardness level. As the concrete matures it becomes harder and harder. The harder the concrete becomes the more resistance is encountered when sawing, and the worse the drift becomes. By adjusting the floor saw speed in dependence of the hardness, the load on the cutting blade can be optimized for a given criterion, such as cutting blade lifetime.

According to aspects, the control unit is arranged to determine a current speed of the floor saw in the forward direction and a desired speed of the floor saw in the forward direction, where the desired speed is determined in dependence of an estimated maturity level or hardness of the concrete surface segment and/or in dependence of a measured temperature of the circular cutting blade, and to control the at least two drive wheels to reduce a difference between the current speed and the desired speed. This way the floor sawing operation can be optimized. For instance, floor sawing speed can be reduced if the cutting blade gets too hot in order to spare the cutting blade and thereby prolong its lifetime. Floor sawing speed can also be configured in dependence of the concrete maturity level or concrete hardness, perhaps such that the speed is reduced when the concrete gets more and more hard in order to spare the blade from cutting too fast in hard concrete. The speed of the floor saw can also be optimized to avoid glazing of the cutting segments on the cutting blade by balancing rotational speed of the cutting blade against forward speed by the floor saw, as will be explained in more detail below.

According to aspects, the control unit is arranged to determine whether an operator is in position to perform manual control of floor saw steering, and to trigger a warning signal and/or halt the floor saw in response to determining that an operator is not performing manual control of the floor saw steering. This feature is similar to a lane assist function in a car, where the floor saw may proceed with cutting in a straight line without manual control as long as an operator is in position to perform manual control, e.g., in case something goes wrong. To be in position to perform manual control may comprise, e.g., an operator having his hands on a control handle or remote control, or simply that an operator is in vicinity of the floor saw, such as in the same room or within a certain distance from the floor saw.

According to aspects, the control unit is arranged to receive concrete maturity data indicating a concrete maturity level of the concrete surface segment from a concrete maturity sensor arranged external to the floor saw, to determine an onset of a suitable time slot for early entry sawing based on the concrete maturity data, and to indicate the onset of the time window to an operator.

Thus, the operator receives assistance in when to start using the floor saw, i.e., when the concrete is in the green zone mentioned above, which is an advantage.

There are also disclosed herein early entry concrete saws as well as more general concrete surface saws, power trowels, and other types of concrete processing equipment, as well as methods associated with the advantages mentioned above.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. Further features of, and advantages with, the present invention will become apparent when studying the appended claims and the following description. The skilled person realizes that different features of the present invention may be combined to create embodiments other than those described in the following, without departing from the scope of the present invention.

### BRIEF DESCRIPTION OF THE DRAWINGS

The present disclosure will now be described in more detail with reference to the appended drawings, where
- Figures 1A,B: show example concrete processing equipment;
- Figure 2: schematically shows a drive arrangement for a floor saw;
- Figure 3: illustrates a remote controlled floor saw;
- Figure 4: shows a concrete processing system;
- Figure 5: is a graph illustrating wheel force vs wheel slip;
- Figure 6: is a graph illustrating concrete maturity as function of time;
- Figure 7: is a flow chart illustrating methods;
- Figure 8: schematically illustrates a control unit;
- Figure 9: schematically illustrates a computer program product;
- Figures 10A,B: illustrate contact force F as function of rotational velocity V;
- Figure 11: schematically illustrates an up-cut based floor saw; and
- Figure 12: schematically shows a control unit.

### DETAILED DESCRIPTION

The invention will now be described more fully hereinafter with reference to the accompanying drawings, in which certain aspects of the invention are shown. This invention may, however, be embodied in many different forms and should not be construed as limited to the embodiments and aspects set forth herein; rather, these embodiments are provided by way of example so that this disclosure will be thorough and complete, and will fully convey the scope of the invention to those skilled in the art. Like numbers refer to like elements throughout the description.

It is to be understood that the present invention is not limited to the embodiments described herein and illustrated in the drawings; rather, the skilled person will recognize that many changes and modifications may be made within the scope of the appended claims.

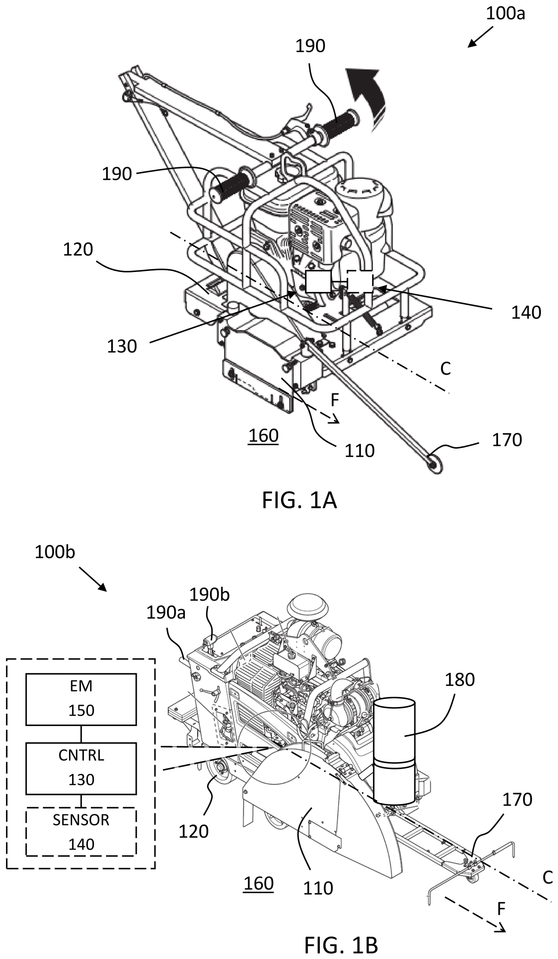

Figures 1A and 1B illustrate early entry concrete floor saws 100a, 100b. As mentioned above, this type of saw is used to cut shallow cuts in concrete surfaces after finishing surface preparation, but before the concrete has set completely.

A concrete surface is associated with a concrete surface segment which comprises the top surface of the concrete and a segment immediately below the surface. The thickness of the concrete surface segment through which sawing is done varies, but is normally below 10-15 cm.

A concrete surface saw, such as an early entry concrete floor saw, generally comprises a circular cutting blade 110 aligned with a forward direction F of the floor saw. Thus, the forward direction is parallel to the cutting blade plane. The floor saw also comprises supporting wheels 120 to support the floor saw against the concrete surface segment 160 to be cut. One or more of the supporting wheels may be driven wheels, which means that they are connected to a propulsion device, such as an electric machine 150, arranged to provide positive torque to the wheel in order to move the saw in the forward direction. On or more of the supporting wheels may also comprise respective wheel brakes arranged to provide negative torque to the wheel. It is appreciated that a floor saw may comprise one or more supporting wheels with respective wheel brakes even if there are no driven wheels, e.g., if the cutting blade rotation is used as sole means for propulsion.

The floor saws discussed herein also comprise a control unit 130 configured to control various functions on the floor saw, such as controlling two or more driven wheels on the floor saw, and/or one or more wheel brakes. The control unit 130 may optionally be connected to one or more sensor devices 140 configured to sense a motion behavior of the floor saw such as a current yaw motion and/or a current speed in the forward direction.

The floor saw generates dust and slurry as a result of the cutting operation. This dust and slurry may be collected in a dust container, such as the Longopac dust bin system which is well known in the art. The dust and slurry is then transported via conduit from the cutting blade area to the dust container by a dust extractor, i.e., a vacuum device in a known manner. This dust extractor can be a separate unit arranged, e.g., to be towed by the floor saw, or it can be integrated with the floor saw. A dust container 180 may, according to some aspects, be mounted on the floor saw, as schematically illustrated in Figure 1B, or comprised in a dust extractor arranged external to the floor saw. In case the floor saw is remote controlled, a signal can be transmitted to the remote control indicating when the dust container has reached its holding capacity, which feature will be discussed in more detail below.

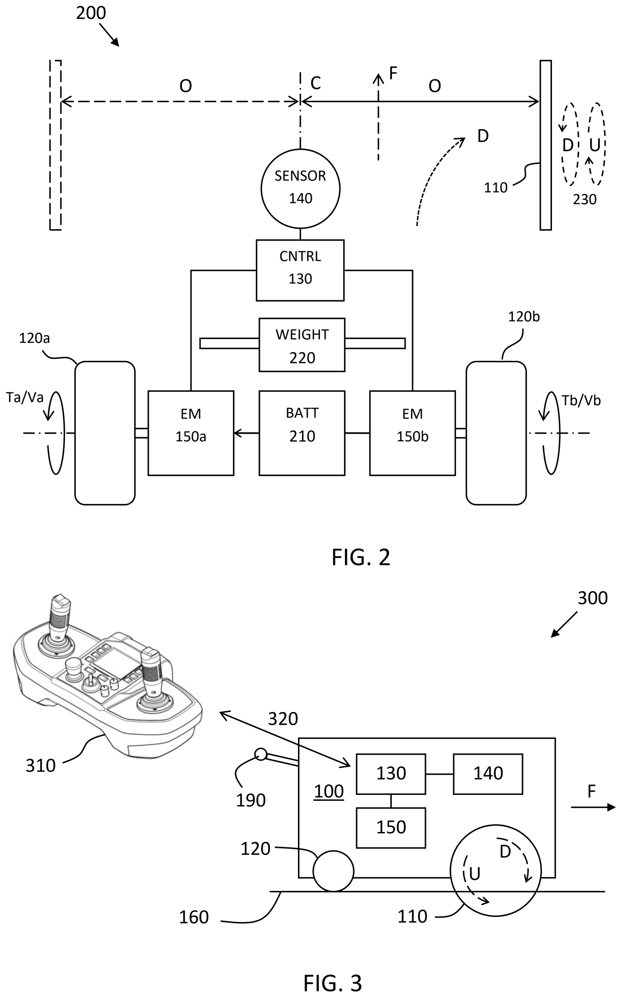

Figure 2 schematically illustrates a drive arrangement for a floor saw 200. The circular cutting blade 110 is offset O to one side of the centrum line C, which centrum line is aligned with the forward direction F. I.e., the centrum line C is parallel with an axis extending in the forward direction. The centrum line is also parallel to the plane defined by the circular cutting blade 110. This offset from the centrum line (and from the mass center of the floor saw) generates a yaw motion by the floor saw during use. Thus, if no countermeasures are taken, the floor saw will drift towards the side with the saw blade, as indicated by the curved arrow D in Figure 2.

Some floor saws allow for mounting the circular cutting blade on either side of the machine, i.e., to the right or to the left of the centrum line C. This has been illustrated in Figure 2 by a dashed line cutting blade on the left hand side. This simplifies reaching certain places close to objects and will of course affect the direction of the drift. It is appreciated that the teachings of the present disclosure are not dependent on which side the cutting blade is located. The sensor arrangements disclosed herein are optionally capable of detecting yaw motion in any direction, and the drive arrangements are optionally configured to compensate for drift in any direction.

The cutting blade 110 may be configured for either down-cut D or up-cut U operation 230. Up-cut operation is common for early-entry saws such as the Soff-cut range of floor saws, while down-cut is more common on regular floor saws. The techniques disclosed herein are applicable for both down-cut as well as up-cut operation.

In some rare cases the floor saw may drift away from the side with the saw blade, i.e., to the left in Figure 2. The techniques disclosed herein can be adapted to compensate for such drifts in a straightforward manner by varying wheel forces to compensate for the drift. Also, as will be understood from the below description, the floor saw can be steered by generating a controlled amount of desired drift to either left or right hand side.

According to some aspects, the floor saw comprises a drive arrangement with one or more separate electrical machines 150a, 150b arranged to control respective supporting wheels 120a, 120b. The electrical machines may be powered by an electrical storage device, such as a battery or a fuel cell arrangement. The electric machines 150a, 150b are arranged to be controlled by the control unit 130 to generate a desired wheel torque Ta, Tb, and/or a desired wheel speed Va, Vb. Thus, it is appreciated that one or more supporting wheels may be driven wheels capable of generating positive torque and therefore a wheel force acting in the forward direction F. Generally an electrical machine can be configured also for generating a negative torque by applying a regenerative braking function. The electrical machines discussed herein may or may not comprise such regenerative braking functions. An electrical machine without regenerative braking function may also be referred to as an electrical motor.

One objective of the present disclosure is to provide floor saws with at least one controllable drive wheel, and preferably two controllable drive wheels, which can be used to dynamically compensate for the drift D, thus providing a floor saw or road saw with a reduced tendency for unwanted drifting towards the side with the cutting blade 110. Towards this end, the saws discussed herein comprise a control unit 130 which controls the drive wheel or wheels to obtain a desired yaw motion by the floor saw. Notably, this desired yaw motion may be a zero yaw motion (corresponding to cutting along a straight line, or a non-zero desired yaw motion (corresponding to cutting along a pre-determined arcuate path).

With reference to Figures 1A and 1B, there is disclosed herein a floor saw 100, 100a, 100b for sawing in a concrete surface segment 160. The floor saw comprises a control unit 130 and a circular cutting blade 110 arranged transversally offset O from a centrum line C of the floor saw, which centrum line C is aligned with a forward direction F of the floor saw. The floor saw further comprising a first drive wheel 120a and a second drive wheel 120b separated by the centrum line C and arranged to support the floor saw on the concrete surface segment 160, where the drive wheels 120a, 120b are arranged to be driven by respective first and second electric machines 150a, 150b. The control unit 130 is arranged to control a difference between wheel forces of the drive wheels by controlling a respective torque and/or speed of the first and/or second electric machines 150a, 150b, in dependence of a desired yaw motion by the floor saw. Thus, the drift of the floor saw due to the offset mounting of the cutting blade can be compensated for in a convenient and efficient manner by varying wheel forces of the two drive wheels. The floor saw can be made to turn both left and right, since the two drive wheels are arranged separated by the centrum line, i.e., are arranged on either side of the floor saw. It is noted that this floor saw control arrangement can be used with or without sensor arrangements that determine a current yaw motion of the floor saw. In case no sensors are present to detect current yaw motion, an operator would need to manually control the yaw motion by the floor saw. In case one or more sensors are arranged on the floor saw to detect current yaw motion, then the control unit can be configured to automatically control floor saw operation to follow a configurable desired yaw motion.

According to some aspects, the floor saw comprises a control input device connected to the control unit 130 and arranged to receive an operator input indicative of the desired yaw motion by the floor saw. Thus, an operator can manually control the yaw motion by the floor saw via the control input device. The control input device may be arranged on a remote control device 310 and/or as an input device on the floor saw. A lever or small steering wheel can, for instance, be arranged on the control handle 190 of the floor saw.

It is appreciated that a cutting blade may not be able to withstand very high yaw motions by the floor saw, since this exerts a strain on the blade. To prevent high wear on the blade, and even blade breakage, the control unit 130 is optionally arranged to limit the current yaw motion of the floor saw to be below a configurable maximum yaw motion amount. This configurable maximum yaw motion amount can, e.g., be automatically configured in dependence of a floor saw cutting blade specification. For instance, a model or other identification of the currently mounted saw blade can be input to the control device, which then determines the maximum allowable yaw motion, e.g., based on an onboard database. According to some aspects, the configurable maximum yaw motion amount corresponds to sawing along a straight line, i.e., the maximum permitted yaw motion amount can in some cases be zero, although normally a positive amount of yaw motion will be permitted.

Some floor saws may be configurable in a transportation mode of operation, where the saw blade is removed from the concrete segment, and where it is desired to relocate the floor saw. In this case the drive wheels can be used to move the floor saw around in a convenient manner. In such saws, the circular cutting blade is optionally configurable in an engaged mode of operation where the circular cutting blade 110 is in position to engage the concrete surface segment 160, and in a disengaged mode of operation where the circular cutting blade 110 is withdrawn from the concrete surface segment 160. The saw blade may, e.g., be arranged to be lifted up from the concrete segment, or the floor saw may comprise a tilting mechanism similar to that disclosed in EP1637655B1. The control unit 130 is then advantageously arranged to limit the current yaw motion of the floor saw to be below the configurable maximum yaw motion amount when the circular cutting blade 110 is in the engaged mode of operation, and to allow a current yaw motion amount larger than the configurable maximum yaw motion amount when the circular cutting blade 110 is in the disengaged mode of operation. This way, the saw blade is protected from strain due to excessive yaw motion when it engages the concrete segment, but the saw is easily maneuvered on the concrete segment when the saw blade is in the disengaged position. As noted above, the yaw motion by the floor saw can be controlled via the control input device connected to the control unit 130 and arranged to receive an operator input indicative of the desired yaw motion by the floor saw, as discussed above. This allows an operator to steer the floor saw during transportation in a convenient manner, e.g., by using a joystick on the remote control or by using a control input device arranged on the machine.

It is again noted that the configurable maximum yaw motion amount may correspond to sawing along a straight line, i.e., the maximum yaw motion amount can in some cases be zero, although normally a positive amount of yaw motion will be permitted. A zero maximum yaw motion amount means that a yaw motion will be permitted by the control unit 130 only when the saw is in the disengaged position, and not in the engaged position. However, normally, an amount of yaw motion is permitted in both the engaged and the disengaged position, although the permitted yaw motion in the disengaged position is often larger than the yaw motion permitted by the control unit 130 in the engaged position.

The control unit 130 may be arranged to detect when the circular cutting blade 110 is configured in the engaged mode of operation by a sensor, such as a switch, arranged to be triggered by the saw blade or by a saw blade holder part in the engaged position. A linear sensor or angle sensor can also be used to determine a working position of the saw blade, i.e., how the saw blade is positioned relative to the concrete surface segment 160. The control unit may also be arranged to detect when the saw blade is in the disengaged position by a similar sensor arrangement. It is, however, noted that the limit on the current yaw motion of the floor saw is normally only active in the engaged mode of operation, hence it is sufficient if the control unit is able to detect when the saw blade is in the engaged position. Of course, if the control unit also actively controls the position of the saw blade relative to the concrete surface segment, by some form of actuator, like a motor and spindle arrangement or a linear actuator, then the control unit can also be configured to indirectly determine the mode of operation by the current control signal sent to the actuator.

This feature is applicable without other features discussed herein. Thus, there is disclosed herein a floor saw 100, 100a, 100b for sawing in a concrete surface segment 160. The floor saw comprises a control unit 130 and a circular cutting blade 110 arranged transversally offset O from a centrum line C of the floor saw, which centrum line C is aligned with a forward direction F of the floor saw. The circular cutting blade 110 is configurable in an engaged mode of operation where the circular cutting blade 110 is in position to engage the concrete surface segment 160, and in a disengaged mode of operation where the circular cutting blade 110 is withdrawn from the concrete surface segment 160. The floor saw further comprises a first drive wheel 120a and a second drive wheel 120b separated by the centrum line C and arranged to support the floor saw on the concrete surface segment 160, where the drive wheels 120a, 120b are arranged to be driven by respective first and second electric machines 150a, 150b. The control unit 130 is arranged to control a difference between wheel forces of the drive wheels by controlling a respective torque and/or speed of the first and/or second electric machines 150a, 150b, in dependence of a desired yaw motion by the floor saw. The control unit 130 is also arranged to limit a yaw motion of the floor saw to be below the configurable maximum yaw motion amount when the circular cutting blade 110 is in the engaged mode of operation, and to allow a yaw motion amount larger than the configurable maximum yaw motion amount when the circular cutting blade 110 is in the disengaged mode of operation.

With reference to at least Figures 1A, 1B and Figure 2, there is also disclosed herein a floor saw 100, 100a, 100b suitable for early entry sawing in a maturing concrete surface segment 160, and also for more general concrete surface sawing operations. The floor saw comprises a circular cutting blade 110 arranged transversally offset O from a centrum line C, which centrum line C is aligned with a forward direction F of the floor saw. The floor saw comprises at least two supporting wheels 120, 120a, 120b arranged to support the floor saw on the concrete surface segment 160, a sensor arrangement 140 configured to determine a current yaw motion of the floor saw, and a control unit 130 configured to obtain a desired yaw motion setting. At least one of the supporting wheels 120, 120a, 120b is arranged on an opposite side of the centrum line C compared to the circular cutting blade 110 and arranged to generate a respective variable wheel force. The control unit 130 is arranged to control a difference between the wheel forces of the supporting wheels, thereby reducing a difference between the current yaw motion of the floor saw and the desired yaw motion setting to provide a drift compensation function.

A yaw motion is herein to be interpreted as a rotation about some point of the floor saw, such as a mass center or a center of inertia. A positive yaw motion is herein defined as a clockwise rotation when seen from above, such as the view in Figure 2. Thus, a positive yaw motion in combination with a forward motion results in an arcuate path to the right as illustrated by the curved arrow D in Figure 2.

A wheel force is the force generated in the longitudinal direction of the floor saw by a supporting wheel, i.e., in the forward direction F or in a reverse direction opposite to the forward direction. With reference to Figure 2, if the wheel 120a generates less force in the forward direction F than the wheel 120b, then a drift D towards the side of the cutting blade 110 will be countered and the floor saw will move with less drift. A wheel force can be generated by applying a torque Ta, Tb, to the respective wheel axles, or generating a wheel speed Va, Vb about the wheel axles. Application of a wheel brake also results in a wheel force, acting in the reverse direction.

It is appreciated that, although most of the present disclosure relates to electric machines arranged to generate the respective variable wheel force, variable wheel forces can also be generated by a combustion engine, or a hydraulic engine. The variation in wheel force for a combustion engine drive can, for instance, be generated by means of separate clutches or variable gear transmission. The variation in wheel force can also be achieved, e.g., by a torque vectoring system, or one or more wheel brakes arranged to selectively brake a wheel to reduce the wheel force generated at this wheel. A variable wheel normal force can also be obtained by arranging a movable weight as will be discussed in more detail below.

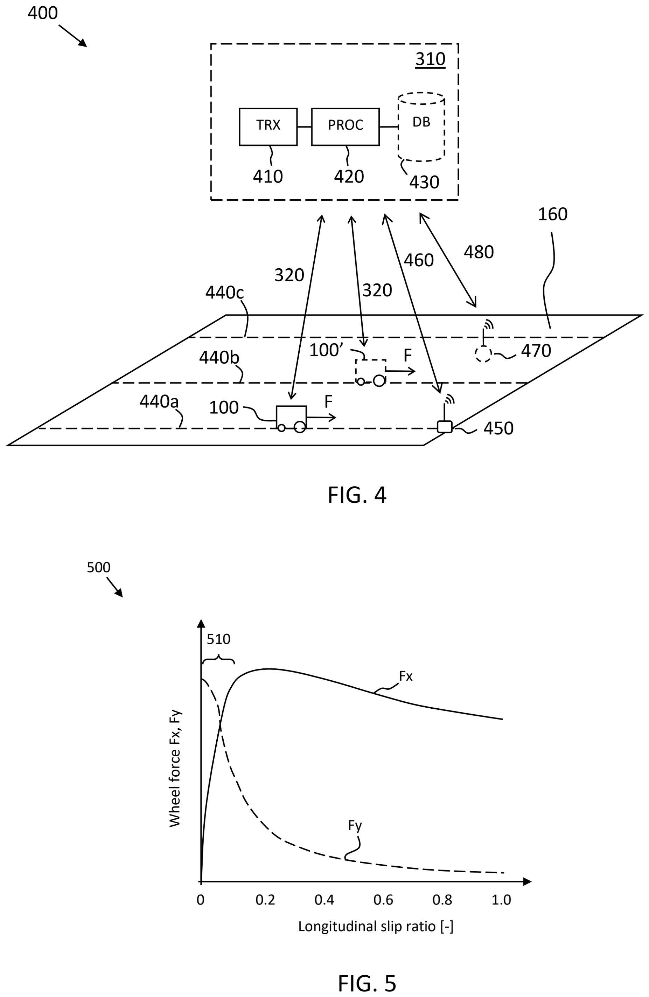

Figure 5 shows a graph 500 illustrating the general relationship between generated wheel force (longitudinal force Fx in the forward direction F and lateral force Fy transversal to the forward direction) and wheel slip, i.e., the difference between wheel rotational speed and the speed of the floor saw. It is seen that a wheel force can be generated by setting a wheel speed higher than the speed of the floor saw to generate a positive wheel slip, at least when the wheel slip is within a range 510 where the relationship is approximately linear. It is also noted that generated lateral force declines quickly with wheel slip. However, such large values of wheel slip are not to be expected in a floor saw.

Generally, it is appreciated that the control unit 130 may be arranged to control the difference between the first wheel force and the second wheel force by controlling respective wheel torques and/or wheel speeds of at least one supporting wheels 120, 120a, 120b. This wheel may be a driven wheel or a wheel with a wheel brake. The wheel force may also be controlled by adjusting a wheel normal load, i.e. the weight distribution between the supporting wheels, as will be discussed in more detail below. The floor saw may for instance as mentioned above comprise a first drive wheel 120a and a second drive wheel 120b, where each drive wheel is driven by a respective first and second electric machine 150a, 150b controlled by the control unit 130. Electric machines like the electric machines 150a, 150b can be controlled by requesting a torque or a wheel speed, both options can be used with the techniques discussed herein for controlling a yaw motion of a floor saw.

The first and the second electric machine may optionally be powered by an on-board electrical storage device 210, such as a battery, a super-capacitor, or a fuel-cell arrangement. This is an advantage since the floor saw can then be transported shorter distances by means of the driven wheels. The electric machines may of course also be powered by cable from electric mains.

The cutting blade on the floor saw may be powered by a separate combustion engine. This engine can then be used to charge the electrical energy source, thus allowing for operating the driven wheels without access to electrical mains or other battery charging means.

A negative wheel torque can be generated by a wheel brake, such as a friction brake. Thus, if a wheel brake is applied at the left wheel 120a, then a drift to the right in Figure 2 will be countered. It is appreciated that the control unit 130 may be arranged to generate variable wheel forces by applying wheel brakes at one or more of the wheels. In other words, a first supporting wheel 120a arranged on an opposite side of the centrum line C compared to the circular cutting blade 110 may be configured with a wheel brake arranged to be controlled by the control unit 130 to generate a negative wheel torque. This negative wheel torque can then be used to counter a drift D towards the side of the circular cutting blade 110.

According to some aspects, the drive arrangement 200 also comprises a weight 220 and an actuator arranged to move the weight transversal to the forward direction F. The actuator may, e.g., be connected to a spindle or the like for moving the weight, and the control unit 130 may then move the weight transversally to the forward direction by the actuator. The weight 220 can be used to shift normal load between the supporting wheels 120a, 120b, thus generating more or less traction on a given wheel and consequently also a different wheel force at each wheel. This increased and/or reduced traction can be used by the control unit to compensate for the drift D.

Figure 2 also shows a sensor arrangement 140. This sensor arrangement 140 is configured to determine the current yaw motion of the floor saw and it can be realized in a number of different ways as will be explained in the following. Of course, combinations of different sensors may also be used to provide more robust and accurate estimates of the current yaw motion. The control unit 130 is configured to receive sensor data from the sensor device 140 and to estimate a current yaw motion from the sensor data. This estimation of current yaw motion can be implemented as a filtering operation, e.g., an averaging operation, or as more advanced signal processing techniques, comprising a Kalman filter or the like.

The sensor arrangement 140 may for instance comprise an inertial measurement unit (IMU) configured to detect the current yaw motion by the floor saw via measurements of acceleration. To improve the acceleration measurement, the IMU is optionally arranged distally mounted on a guiding arm 170 extending in the forward direction F, as shown in Figures 1A and 1B. The IMU will most likely be subject to significant vibration and perhaps also shock, which implies that the sensor data from an IMU may need to be heavily filtered to extract the current yaw motion in order to suppress effects from vibration. The IMU can optionally also be mounted on a resilient member configured to suppress vibrations.

The sensor arrangement 140 may also comprise one or more vision-based sensors configured to detect the current yaw motion by the floor saw. This can be done, for instance, by sequentially recording scenes in the forward direction, and correlating two or more consecutive recorded scenes with each other to detect offsets to the left or to the right, which offsets then indicate a drift and a yaw motion. Optionally, a scaling operation can be performed on the recorded scenes to compensate for the forward motion by the floor saw. However, if the scenes are recorded frequently enough, such as at a rate of a couple of Hz or so, then no such scaling should be necessary. If a given object continuously shifts to the left in a captured sequence of images, the floor saw is assumed to be drifting to the right. It is also possible to deploy some visual marker (such as a signpost or the like) which a vision-based sensor can locate in the captured images and track over time to detect a yaw motion by the floor saw. The control unit may also be configured to navigate towards a deployed visual mark and thereby generate a cut along a straight line from an initial position towards the visual mark.

Various wireless signals can also be used to detect a yaw motion. For instance, the sensor arrangement 140 may comprise a receiver configured to receive a wireless signal from a beacon 450, and to detect the current yaw motion by the floor saw and/or a current heading of the floor saw based on the signal. An RF beacon can be used together with a directive antenna to detect if the floor saw is veering off course, which will be detected by a reduced received signal power. An infrared (IR) beacon can be used together with an IR sensor to detect a relationship between a course of the floor saw and a direction of the IR beacon. A yaw motion can be detected by comparing the bearing to the IR beacon to the floor saw over time.

The sensor arrangement 140 may of course also comprise an electronic compass configured to measure the forward direction F of the floor saw in relation to magnetic north. The control unit 130 can then be configured to determine the current yaw motion of the floor saw based on measurements of the forward direction F in relation to magnetic north over time. If the compass device is accurate enough, an operator can set a course to be followed by the machine. The machine can then be placed in a semi-autonomous mode to complete a cut over a configured length, where the length of the cut can be determined based on dead reckoning, based on an indoor positioning system, or the like.

According to some aspects, the floor saw comprises a radar sensor or an ultrasound sensor configured to detect obstacles in front of the floor saw. The floor saw can then be controlled by the control unit 130 to cut in a certain direction until it reaches an obstacle such as a wall or the like, whereupon the floor saw automatically halts in response to a signal from the radar or ultrasound sensor. Of course, a stereo vision sensor can also be used to detect distances to obstacles in the forward direction.

Figure 3 illustrates a floor sawing system 300 comprising a floor saw and a remote control device 310. The control unit 130 is here arranged to receive the desired yaw motion setting via wireless radio link 320 from the remote control device 310. Thus, an operator may steer the floor saw via the remote control. The steering may just be a command to start sawing along a straight line with no drift, or to saw along some arcuate path, perhaps specified by a radius configured on the remote control device 310. In other words, the remote control device 310 is optionally configured to transmit a desired yaw motion setting corresponding to sawing along a straight line or corresponding to sawing along an arcuate curve having a configurable radius.

The operator may also wish to generate a cut at a deviation from a defined direction or path. The machine may then be configured to execute a controlled turning maneuver along an arcuate path with a radius that does not jeopardize the cutting blade and to continue cutting along the new direction after the deviation has been reached.

Figure 4 schematically illustrates a concrete processing system where one or more floor saws 100, 100' are used to make early entry cuts 440a, 440b, 440c in a concrete surface segment 160. The one or more floor saws 100, 100' are connected via wireless link 320 to the remote control device 310. Thus, the remote control device 310 may be configured to control one or more further floor saws 100 in addition to the floor saw. The remote control device may comprise a selector for selecting a floor saw in a fleet of floor saws, and controlling the selected floor saw to perform a sawing task.

The remote control device here comprises a transceiver unit 410 to communicate via the wireless link 320 to the one or more floor saws 100, 100', and a processing unit 420, as well as an optional database 430.

Figure 4 shows an example beacon 450 towards which one of the floor saws is steering in an autonomous or a semi-autonomous manner. This beacon may also be connected via wireless link 460 to the remote control device 310. This way an operator may activate beacons selectively so as to not confuse sensor devices on the different floor saws operating in the area. Thus, the remote control device 310 may be arranged to remotely control one or more of the beacons discussed above, i.e., one or more RF beacons or one or more IR beacons.

Figure 4 also shows a concrete maturity sensor 470 arranged to continuously measure moisture and/or temperature in the concrete slab. This maturity data can be used to optimize floor saw control of both cutting speed and drift compensation, as will be discussed in more detail below.

It is appreciated that concrete slabs become harder as they mature. Thus, a concrete maturity level can be translated into a concrete hardness level and vice versa. This type of conversion can be made using a look-up table or the like. It is appreciated that the translation between maturity and hardness may be dependent on the type of concrete, i.e., on the concrete recipe. Thus, it is appreciated that concrete maturity level and concrete hardness are often at least approximately equivalent in terms of information content.

Several different measures of concrete hardness are known, such as the Mohs scale. Hardness is seen as an "attribute" of objects that are difficult to physically alter when subjected to different forms of deformation. Fully cured concrete is a relatively hard material, as its hardness varies between 3 and 7 Mohs. Although this measurement does not indicate anything relevant to concrete, it bears a remarkable relationship to its strength. Hardness is not a true property of materials, since it depends on certain properties of a material, such as ductility, resistance, rigidity, elasticity, viscosity, deformation, among others. Rather, it is a property that is attributed to any object capable of resisting change when it is subject to abrasion or scratching. Objects such as wood, which can be easily scratched, have a lower hardness compared to steel or granite, since it is difficult to scratch them. Hardness of a concrete surface segment can be measured using a non-destructive test which consists of evaluating the impact resistance of a concrete structural element. To perform it, an instrument called Schmidt's hammer, also known as a sclerometer, is used. A scratch test can also be performed, which consists of scratching the concrete surface with a series of 4 pencils, each one with a standardized point and calibrated according to the Mohs scale. The measure of concrete hardness used has no significant impact on the methods and techniques disclosed herein. A simple scale without unit from, say 0-100, is sufficient for most of the features discussed herein.

According to some aspects, the processing unit 420 in the control unit 130 is arranged to receive concrete maturity data indicating a concrete maturity level of the concrete surface segment 160 from the one or more concrete maturity sensors 470 embedded in the concrete surface segment 160, and to adjust a control loop parameter of the control unit for driving the at least two drive wheels in dependence of the concrete maturity data. The control loop adjustment can be done continuously or at least regularly in order to optimize operation to the current conditions. Given a concrete maturity level, and potentially also a concrete recipe comprising the concrete composition and any additives, stored in the database 430 or received from an external source, an ideal floor saw speed in the forward direction F can be determined by consulting the database 430. The control unit 130 may then configure the drive wheels on the floor saw to move the floor in the ideal floor saw speed. This results in a cutting speed which is near optimal for the circular cutting blade 110, thereby minimizing cutting blade wear. It is appreciated that this feature can be implemented in combination with other features discussed herein or independently from the other features discussed herein.

The sensor data reported over the first communication links 480 to the data processing system 310 by the concrete sensors 470 indicates a current maturity state of the concrete slab. However, in some situations it may be advantageous to be able to estimate a future maturity state of a concrete slab.

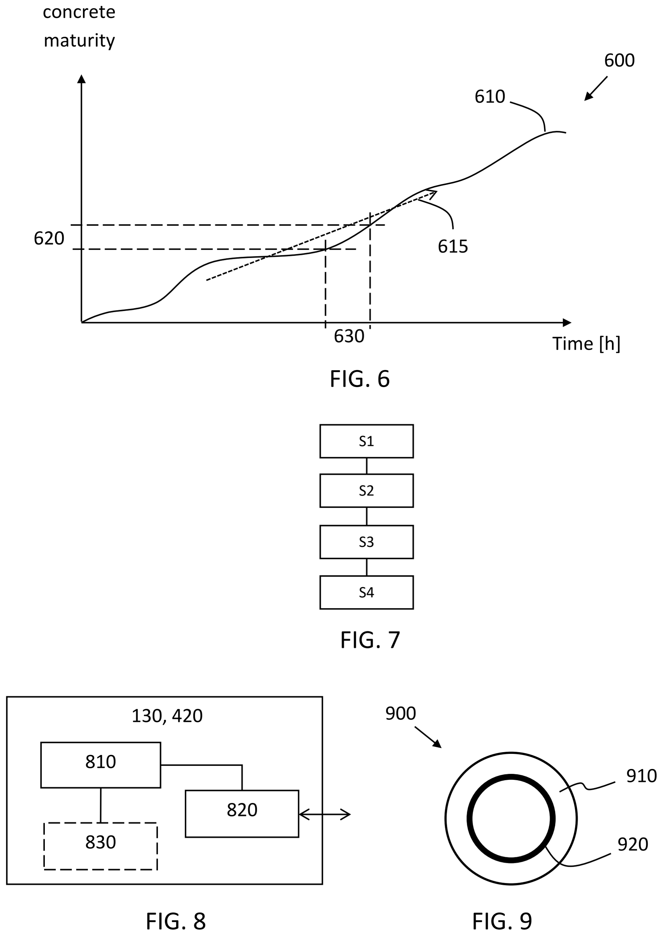

Figure 6 is a graph 600 which illustrates concrete maturity 610 over time, on a time scale of several hours. Concrete maturity can be estimated from sensor data, e.g., as a percentage relative to total maturity or to some other reference value. The maturing process is seldom an entirely linear process. Instead, the rate of change often varies over time. Nevertheless, its rate of change can be estimated, e.g., by low-pass filtering the sensor data or by applying more advanced signal processing operations such as a Kalman filter configured to estimate the evolution over time of the concrete maturity, e.g., as an average rate of change 615. The control units 130 discussed herein may be configured to generate extrapolated temperature and/or moisture level data from the concrete maturity sensor data, and to predict a future onset and/or a future cessation of the time slot for early entry sawing. Thus, a contractor can receive information of an upcoming time slot for early entry sawing, thus greatly simplifying planning.

A given floor saw may be associated with a first range 620 of concrete maturity when processing is at its best, this range corresponds to a first time window 630 with an onset and a cessation time instant. It is appreciated that the onset time instant and the cessation time instant are not necessarily determined on a second-basis. Rather the onset and cessation can be given in more approximate terms, e.g., on a half-hour basis or the like.

Another floor saw may be associated with another ideal range of concrete maturity (possibly even overlapping the first range 620), this range then corresponds to a second time window.

The maturity values corresponding to the ideal time slots for early entry sawing with a given type of floor saw can be stored in the control unit 130 and/or in the database 430 in the control unit. Thus, an operator may configure which floor saw is to be used, and possibly also which circular cutting blade that is currently mounted on the saw, and then receive information regarding the ideal time slot for early entry sawing. According to an example, a visual indicator can be arranged as a display of the remote control device 310 or floor saw which indicates when it is time to start early entry sawing in a given area. This display may also indicate a future point in time when the time slot is estimated to start. The visual indicator may also be less advanced, e.g., just a green light arranged on the floor saw, which is activated by the control unit 130 during the time slot for ideal early entry sawing.

The harder the concrete is, the stronger the longitudinal force generated by the circular cutting blade 110 due to the increased resistance from the concrete. Thus, the database 430 or an equivalent database comprised in the control unit 130 may comprise information which allows to translate between a given drift or drift compensation and a concrete maturity level or measure of concrete hardness. The control unit 130 may thus be arranged to determine a level of drift compensation applied via the at least two drive wheels 120, 120a, 120b to reduce the difference between the current yaw motion of the floor saw and the desired yaw motion setting. The control unit 130 can then determine a concrete hardness and/or concrete maturity level in dependence of the determined level of drift compensation and configure a desired speed of the floor saw in the forward direction F in dependence of the hardness and/or concrete maturity level. Thus, minimizing tool wear or maximizing cutting speed, or any trade-off there in-between, can be achieved by maintaining an ideal cutting speed, i.e., machine speed in the forward direction F, in dependence of the concrete maturity level.

Glazing refers to an effect where the abrasive cutting segments on the cutting blade become dull and stop cutting. Glazing occurs when the cutting segment matrix holding the abrasive particles overheat and cover the abrading particles, i.e., the diamonds. The risk of glazing is a function of the applied cutting blade contact pressure, i.e., the force with which the cutting blade engages the concrete to be cut and the rotation velocity of the cutting segments on the blade 110. The contact pressure can be varied by adjusting wheel forces, and the rotational velocity of the cutting blade can be controlled by, e.g., adjusting the cutting blade engine throttle. In particular, the risk of glazing increases if the cutting blade is operated at high rotational velocity and low contact pressure. With higher contact pressure, a larger rotational velocity can normally be tolerated and vice versa. This means that there is an undesired operating region where the risk of glazing is increased. The size and shape of this undesired operating region depends on the type of cutting segment and on the material to be cut, i.e., the maturity level and recipe of the concrete. This undesired operating region may be configured in a memory accessible by the control unit 130, and the control unit may then control the floor saw wheel forces and the rotational velocity of the cutting blade to avoid operation in the undesired operating region.

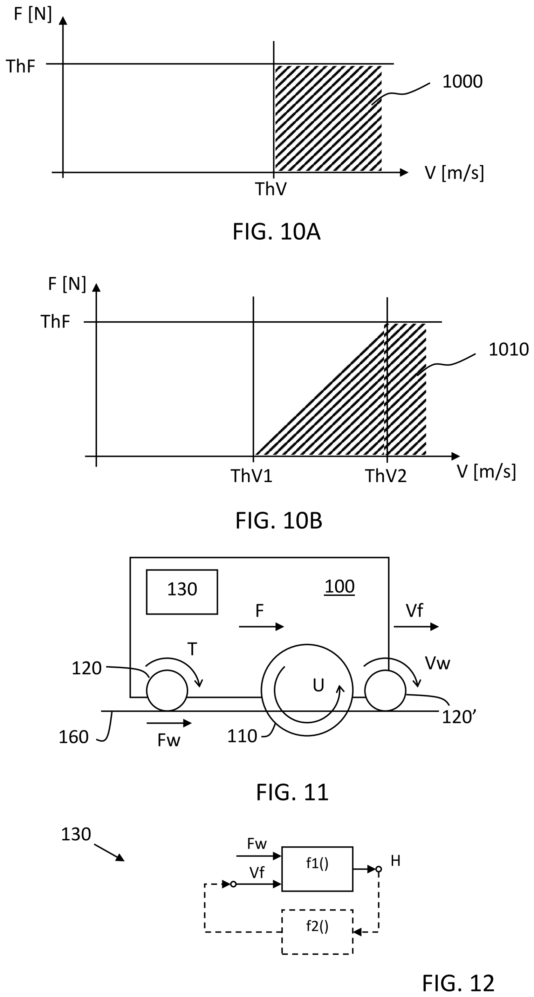

According to a first example, illustrated in Figure 10A, the undesired operating region 1000 is determined by two thresholds; A rotation velocity threshold ThV and a contact force threshold ThF. In this case it is not desired to operate the floor saw for prolonged periods of time above ThV and below ThF. In case a rotational velocity above ThV is desired, then the contact force F should be increased to a value above ThF.

According to a second example, illustrated in Figure 10B, the undesired operating region 1010 starts at a first rotational velocity value ThV1 where the corresponding undesired contact force F increases gradually up to a threshold value ThF at a corresponding tangential velocity value ThV2.

Each circular cutting blade 110 may be associated with a desired rotation speed range or even an optimal rotation speed as function of cutting blade contact pressure. Each blade is normally associated with a preferred range of contact pressure and corresponding blade rotation speed. The techniques disclosed herein allow for estimating a concrete maturity level, and from there a concrete hardness, which estimate can optionally be refined by also taking the concrete recipe into account. Given the concrete hardness, the forward drive speed and/or wheel forces of the floor saw can be configured together with the rotation speed of the cutting blade to obtain a desired cutting characteristic outside of the undesired operating region or even at an optimal combination of blade rotation speed and contact pressure.

It is understood that the optimality criterion can be based on either cutting speed, i.e., the floor saw speed in the forward direction, or cutting blade lifetime, or anywhere in-between. Thus, an operator can configure the desired optimality criterion, and the floor saw control unit 130 can then adjust the forward drive speed and blade rotation speed to optimize performance against the optimality criterion.

This way the tool lifetime can be extended, which is an advantage. Alternatively, the cutting speed, i.e., the floor saw speed in the forward direction, can be improved, which is also an advantage.

There is also disclosed herein a floor saw 100, 100a, 100b for sawing in a concrete surface segment 160. The floor saw comprises a circular cutting blade 110 arranged transversally offset O from a centrum line C, which centrum line C is aligned with a forward direction F of the floor saw. The floor saw further comprises at least two supporting wheels 120, 120a, 120b arranged to support the floor saw on the concrete surface segment 160, wherein the supporting wheels 120, 120a, 120b are arranged to generate a variable floor saw speed in the forward direction. The floor saw further comprises a control unit 130 arranged to determine a measure of concrete hardness of the maturing concrete surface segment, e.g. based on an estimated maturity level of the concrete, and to configure a desired speed of the floor saw in the forward direction F in dependence of the concrete hardness. The control unit 130 is optionally arranged to determine the measure of concrete hardness at least of the maturing concrete surface segment based on any of: a level of drift by the floor saw, a type or recipe of the concrete in the concrete surface segment and/or a concrete maturity level.

With reference to Figure 1 and Figure 2, there is also disclosed herein a floor saw 100, 100a, 100b for sawing in a concrete surface segment 160. The floor saw comprises a circular cutting blade 110 arranged transversally offset O from a centrum line C, which centrum line C is aligned with a forward direction F of the floor saw. The floor saw further comprises at least two supporting wheels 120, 120a, 120b arranged to support the floor saw on the concrete surface segment 160, wherein the supporting wheels 120, 120a, 120b are arranged to generate a variable floor saw speed in the forward direction. The floor saw further comprises a control unit 130 arranged to determine a measure of concrete hardness of the maturing concrete surface segment, and to configure a desired speed of the floor saw in the forward direction F in dependence of the concrete hardness. Thus, in line with the discussion above, this floor saw is able to optimize the cutting speed in the forward direction based on the determined concrete hardness. The desired speed corresponding to a given concrete hardness may be obtained, e.g., from a look up table or pre-determined function. The look-up table may be populated, e.g., by experimental data from one or more trials. It is appreciated that this particular feature may be implemented as a stand-alone feature. For instance, the configuration of the desired speed is not dependent on any drift compensation function, although the two features can be advantageously combined into a floor saw with automatic speed control and drift prevention.

According to aspects, the control unit 130 is arranged to determine the measure of concrete hardness of the maturing concrete surface segment based on any of: a level of drift by the floor saw, a type or recipe of the concrete in the concrete surface segment and/or a concrete maturity level.

It is an advantage that this variation in forward speed is continuously adjusted in dependence of the concrete hardness, thereby adjusting cutting operation to the current concrete hardness.

With reference to Figure 11, there is shown a floor saw 100 according to at least some of the aspects discussed above. The floor saw is arranged for up-cut operation U. The cutting blade therefore acts as a resistance which the driven supporting wheels 120 must overcome by a wheel force Fw in order to generate a speed Vf in the forward direction F. The speed in the forward direction of course corresponds to a speed of the cutting blade through the concrete surface segment. The relationship between wheel force Fw and speed in the forward direction Vf can be used to determine an approximate concrete hardness level. The harder the concrete becomes, the more resistance is encountered by the circular cutting blade, and the more wheel force is required to maintain a desired cutting speed. For instance, data regarding estimated concrete hardness level can be gathered in a matrix indexed by wheel force Fw and speed in the forward direction Vf. Thus, given a wheel force value and a speed, a corresponding concrete hardness level can be obtained. Different such matrixes can be generated for different types of concrete to further improve the estimate of concrete hardness.

Thus, the control unit 130 in Figure 11 may, according to some aspects, be arranged to determine the current speed of the floor saw 100 in the forward direction Vf indicating a speed of the circular cutting blade 110 through the concrete surface segment 160. This speed can, e.g., be determined by a free rolling supporting wheel 120' via its rotational velocity Vw and its radius, by a radar sensor arranged on the floor saw, or by some form of positioning system, such as a global positioning system (GPS) receiver or an indoor positioning system based on deployed beacons.

The control unit 130 is also arranged to determine the wheel force Fw generated by the supporting wheels 120 in the forward direction. This can be done, e.g., by measuring wheel slip as discussed in connection to Figure 5, i.e., the normalized difference in speed between the wheel rotation and the floor saw relative to the concrete surface, or more simply by measuring generated torque T by the motor driving the supporting driven wheels 120, or even more simply by measuring current or power drawn by an electric motor.

With reference also to Figure 12, the control unit 130 is, according to some aspects, configured to determine the concrete hardness level H based on the wheel force Fw and on the current speed in the forward direction Vf. This can be done via a predefined function f1(), which may be in the form of an empirical look-up table as discussed above. The function f1() can also be an analytical or semi-analytical function determined based on, e.g., computer simulation. By determining or estimating the wheel force Fw, and the speed of the cutting blade Vf through the concrete, a measure of concrete hardness level can be obtained. This hardness level can then be used to optimize operations as discussed above.

The hardness level H can be taken as input to a second function f2() which is configured to determine a desired velocity Vf based on the hardness. Thus a feedback loop is closed which controls the cutting speed of the floor saw in dependence of the concrete hardness level. This means that, as the concrete matures, the forward speed is adjusted to account for the variation in concrete hardness level.

The control unit 130 is optionally further arranged to configure a rotational velocity of the circular cutting blade 110 in dependence of the concrete hardness level and of the current speed in the forward direction Vf. This way cutting performance can be optimized to, e.g., avoid glazing as discussed in connection to Figures 10A and 10B, or to maximize the lifetime of the cutting blade, or to maximize the cutting speed, i.e., the speed Vf.

The control unit 130 may also arranged to obtain data indicating a specification of the circular cutting blade 110, and to configure the desired speed of the floor saw in the forward direction F in dependence of the specification. The data may, e.g., be manually input by an operator, or read from a wireless identification tag on the circular cutting blade. This data can, for instance, indicate a type of cutting segments mounted on the circular cutting blade 110, which can be used to determine a preferred contact pressure (which is proportional to the wheel force Fw), and a rotational velocity of the cutting blade 110.

It is appreciated that these features can be implemented in combination with other features discussed herein or independently from the other features discussed herein.

According to some aspects, the control unit 130 is arranged to determine a current speed of the floor saw in the forward direction F and a desired speed of the floor saw in the forward direction F. The desired speed is determined in dependence of an estimated maturity level and/or hardness level of the concrete surface segment 160 and/or in dependence of a measured temperature of the circular cutting blade 110. The control unit is also configured to control the at least two drive wheels 120, 120a, 120b to reduce a difference between the current speed and the desired speed. It is appreciated that this feature is independent of the other features discussed above, i.e., this feature can be implemented as a stand-alone feature without requiring any modification to the other features of the floor saw.

According to other aspects, the control unit 130 is arranged to receive concrete maturity data indicating a concrete maturity level of the concrete surface segment 160 from an external concrete maturity sensor 470, to determine an onset of a suitable time slot for early entry sawing, i.e., the green zone discussed above, based on the concrete maturity data, and to indicate the onset of the time window to an operator. The indication may, e.g., be conveniently transmitted to the remote control device 310 via the wireless link 320. The indication may also comprise activating a visual indicator on the floor saw, such as a green light. A red light on the floor saw can be activated outside of the suitable time slot. It is appreciated that this feature can be implemented in combination with other features discussed herein or independently from the other features discussed herein.

The floor saws discussed herein may furthermore comprise an arrangement configured to determine a weight and/or volume of accumulated dust or slurry in a dust container 180 mounted on the floor saw as discussed above in connection to Figure 1B. The control unit 130 may be arranged to detect when the weight and/or volume of accumulated dust or slurry exceeds a preconfigured threshold and to transmit a signal to the remote control device 310 indicating that the weight and/or volume of accumulated dust or slurry is above the preconfigured threshold. This way, an operator having the remote control device 310 receives an indication when one of the floor saws has accumulated enough dust so as to warrant emptying the dust container. The operator can then stop the floor saw and empty the dust container. The operator then does not have to continuously monitor the dust containers to see if they are full, since he receives the indication remotely. It is appreciated that this feature can be implemented independently of the other features discussed herein. Thus, the signal indicating that the weight and/or volume of accumulated dust or slurry in a dust container is above a preconfigured threshold can be sent to a remote control even if the floor saw does not implement, e.g., drift compensation.

Any weight measurement device can be used to determine the weight of collected dust and slurry, such as electronic or mechanical scales arranged to measure a weight of the dust container 180.

A rudimentary implementation may also be based on a simple timer, which will trigger generation of the signal indicating that the weight and/or volume of accumulated dust or slurry in the dust container is above a preconfigured threshold when the floor saw has been operated for a configurable period of time.

A radar transceiver can also be arranged inside the dust container and arranged to measure free volume inside the dust container.

The different types of sensors can be connected to the control unit, which is then able to determine the weight and/or volume of the collected dust and slurry inside the dust container.

The floor saws in Figures 1A and 1B can be manually operated by an operator via respective control handles 190. Alternatively, as was discussed above in connection to Figure 3, the floor saws may be arranged for remote control. A control handle is herein to be interpreted as a physical device which the operator uses to manually control the floor saw. One example of a control handle are the handle bars 190 shown in Figure 1A which the operator can use to manually guide the floor saw, i.e., determine direction and also in some cases the forward speed by manually holding the floor saw back or pushing the floor saw forward.

A control handle may also be one or both of the control handles 190a and 190b shown in Figure 1B. Control handle 190a can be used to guide the machine, while control handle 190b is a control device used to control one or more operations of the floor saw, such as a forward velocity.

Known methods exist for determining when an operator makes physical contact with a control handle, i.e., when an operator has his hands on the control handle or not. Examples of such sensor systems comprise capacitive sensors which detect presence of the operator's hands, sometimes referred to as "hands-off" sensors. There are also known solutions based on torque sensors which sense small operator inputs to the manual control handles. If no such input is detected for a given time period, then it is determined that the operator is not in a position to manually control the floor saw. The given period of time may either be very small, such that the system immediately triggers upon the operator removing his hands from the control handle, or of some short time duration. For instance, the operator may be allowed to remove his hands from the control handle for a period of a few seconds, such as five seconds, but not longer.

The resemblance to known lane assist advanced driver assistance (ADAS) systems is noted, where the driver is often allowed to let go of the steering wheel for some predetermined duration of time until a warning is issued. Some vehicles such as certain cars are equipped with 'pilot assist' or 'lane keeping' systems which assist a driver by maintaining the vehicle in the current lane. However, for safety reasons, the driver always has to maintain manual control the vehicle, e.g., by regularly moving the steering wheel, or keeping at least one hand on the steering wheel. The floor saws discussed herein may be arranged to perform a similar function. Thus, an operator may be required to perform manual control of, e.g., the steering of the floor saw, at least intermittently. In-between the floor saw may operate in a near autonomous mode, e.g., by going in a straight path towards a beacon as discussed above. Consequently, according to some aspects, the control unit 130 is arranged to determine whether an operator is performing manual control of floor saw steering and/or is in a position to perform manual control with short notice, and to trigger a warning signal and/or halt the floor saw in response to determining that an operator is not performing manual control of the floor saw steering. This also encompasses detecting if an operator is at all present close to the floor saw, e.g., based on camera sensors or radar sensors.

It is appreciated that these features are independent of the other features discussed above, i.e., this feature can be implemented as a stand-alone feature without requiring any modification to the other features of the floor saw.

Figure 7 is a flow chart illustrating methods. There is illustrated a method for controlling operation of a floor saw 100, 100a, 100b for sawing in a concrete surface segment 160. The methods comprises providing S1 a floor saw comprising a circular cutting blade 110 arranged transversally offset O from a centrum line C, which centrum line C is aligned with a forward direction F of the floor saw, wherein the floor saw further comprises at least two supporting wheels 120, 120a, 120b arranged to support the floor saw on the concrete surface segment 160 wherein at least one of the supporting wheels 120, 120a, 120b is arranged on an opposite side of the centrum line C compared to the circular cutting blade 110 and arranged to generate a respective variable wheel force. The method also comprises determining S2 a current yaw motion of the floor saw, and obtaining S3 a desired yaw motion setting, and controlling S4 a difference between the wheel forces of the supporting wheels to reduce a difference between the current yaw motion of the floor saw and the desired yaw motion setting.

Figure 8 schematically illustrates, in terms of a number of functional units, the general components of a control unit 130, 420. Processing circuitry 810 is provided using any combination of one or more of a suitable central processing unit CPU, multiprocessor, microcontroller, digital signal processor DSP, etc., capable of executing software instructions stored in a computer program product, e.g. in the form of a storage medium 830. The processing circuitry 810 may further be provided as at least one application specific integrated circuit ASIC, or field programmable gate array FPGA.

Particularly, the processing circuitry 810 is configured to cause the device 180 to perform a set of operations, or steps, such as the methods discussed in connection to Figure 6 and the discussions above. For example, the storage medium 830 may store the set of operations, and the processing circuitry 810 may be configured to retrieve the set of operations from the storage medium 830 to cause the device to perform the set of operations. The set of operations may be provided as a set of executable instructions. Thus, the processing circuitry 810 is thereby arranged to execute methods as herein disclosed.

The storage medium 830 may also comprise persistent storage, which, for example, can be any single one or combination of magnetic memory, optical memory, solid state memory or even remotely mounted memory.

The device 130, 420 may further comprise an interface 820 for communications with at least one external device. As such the interface 820 may comprise one or more transmitters and receivers, comprising analogue and digital components and a suitable number of ports for wireline or wireless communication.

The processing circuitry 810 controls the general operation of the control unit 130, 420, e.g., by sending data and control signals to the interface 820 and the storage medium 830, by receiving data and reports from the interface 820, and by retrieving data and instructions from the storage medium 830.

Figure 9 illustrates a computer readable medium 910 carrying a computer program comprising program code means 920 for performing the methods illustrated in Figure 7, when said program product is run on a computer. The computer readable medium and the code means may together form a computer program product 900.

## Claims

1. A floor saw (100, 100a, 100b) comprising a control unit (130) and an arrangement configured to determine a weight and/or volume of accumulated dust or slurry in a dust container (180) mounted on the floor saw, **characterized in that** the control unit (130) is arranged to detect when the weight and/or volume of accumulated dust or slurry exceeds a preconfigured threshold and to transmit a signal to a remote control device (310) indicating that the weight and/or volume of accumulated dust or slurry is above the preconfigured threshold.

2. The floor saw (100, 100a, 100b) according to claim 1, comprising a weight measurement device arranged to determine the weight of accumulated dust or slurry, such as electronic or mechanical scales arranged to measure a weight of the dust container (180).

3. The floor saw (100, 100a, 100b) according to claim 1 or 2, comprising a timer configured to trigger generation of the signal indicating that the weight and/or volume of accumulated dust or slurry is above the preconfigured threshold when the floor saw has been operated for a configurable period of time.

4. The floor saw (100, 100a, 100b) according to any previous claim, comprising a radar transceiver arranged inside the dust container to measure a free volume inside the dust container, where the radar transceiver is connected to the control unit (130) which is configured to determine the weight and/or volume of the collected dust and slurry inside the dust container based on the radar measurement.

5. The floor saw (100, 100a, 100b) according to any previous claim, comprising a circular cutting blade (110) arranged transversally offset (O) from a centrum line (C), which centrum line (C) is aligned with a forward direction (F) of the floor saw, the floor saw further comprising at least two supporting wheels (120, 120a, 120b) arranged to support the floor saw on the concrete surface segment (160), a sensor arrangement (140) configured to determine a current yaw motion of the floor saw, and the control unit (130) configured to obtain a desired yaw motion setting,
wherein at least one of the supporting wheels (120, 120a, 120b) is arranged on an opposite side of the centrum line (C) compared to the circular cutting blade (110) and arranged to generate a respective variable wheel force, and
wherein the control unit (130) is arranged to control a difference between the wheel forces of the supporting wheels to reduce a difference between the current yaw motion of the floor saw and the desired yaw motion setting.

6. The floor saw (100, 100a, 100b) according to claim 5, wherein the at least two supporting wheels (120, 120a, 120b) are driven wheels, and wherein the control unit (130) is arranged to control the difference between the wheel forces of the supporting wheels by controlling respective wheel torques and/or speeds of the at least two driven wheels (120, 120a, 120b).

7. The floor saw (100, 100a, 100b) according to any previous claim, wherein the supporting wheels (120, 120a, 120b) comprising a first drive wheel (120a) and a second drive wheel (120b), where each drive wheel is driven by a respective first and second electric machine (150a, 150b) controlled by the control unit (130).

8. The floor saw (100, 100a, 100b) according to claim 7, wherein the first and second electric machine is arranged to be powered by an on-board electrical storage device (210).

9. The floor saw (100, 100a, 100b) according to claim 2, wherein the control unit (130) is arranged to receive the desired yaw motion setting via wireless radio link (320) from the remote control device (310).

10. A remote control device (310) arranged to receive a signal from one or more floor saws (100, 100a, 100b) according to any previous claim, **characterized in that** the received signal indicates that a weight of accumulated dust or slurry in one of the one or more floor saws is above a preconfigured threshold, and **in that** the remote control device (310) is configured to notify an operator in case the weight of accumulated dust or slurry is above the preconfigured threshold.

11. The remote control device (310) according to claim 10, comprising a selector for selecting a floor saw out of the one or more floor saws (100, 100a, 100b), where the remote control device (310) is configured to control the selected floor saw to perform a sawing task.

12. The remote control device (310) according to claim 10 or 11, arranged to receive the signal via wireless link to the one or more floor saws (100, 100a, 100b).

13. A method performed by a control unit (130) in a floor saw (100, 100a, 100b) comprising an arrangement configured to determine a weight and/or volume of accumulated dust or slurry in a dust container (180) mounted on the floor saw,
the method comprising
configuring a threshold of accumulated dust or slurry,
detecting when the weight and/or volume of accumulated dust or slurry exceeds the preconfigured threshold, and
transmitting a signal to a remote control device (310) of the floor saw (100, 100a, 100b) indicating that the weight and/or volume of accumulated dust or slurry is above the preconfigured threshold.
